# EUROPEAN PATENT SPECIFICATION

(11) **EP 3 487 368 B1**
(45) Date of publication and mention of the grant of the patent: **04.03.2020**
(21) Application number: 17742998.2
(22) Date of filing: 14.07.2017
(51) Int. Cl.: A47J 43/07

(54) **FOOD BLENDER AND BLENDING METHOD**
LEBENSMITTELMIXER UND MIXVERFAHREN
MÉLANGEUR DE NOURRITURE ET PROCÉDÉ DE MÉLANGE

(30) Priority: 25.07.2016 WO PCT/CN2016/091534; 20.09.2016 EP 16189675
(43) Date of publication of application: 29.05.2019
(73) Proprietor: Koninklijke Philips N.V., 5656 AE Eindhoven (NL)
(72) Inventor: BAO, Weishun, 5656 AE Eindhoven (NL); XIAO, Weimin, 5656 AE Eindhoven (NL); LU, Weihua, 5656 AE Eindhoven (NL); JIN, Yafang, 5656 AE Eindhoven (NL)
(74) Representative: Philips Intellectual Property & Standards
(86) International application number: PCT/EP2017/067891
(87) International publication number: WO 2018/019620

(56) References cited:
- WO-A1-2016/072203
- WO-A1-2016/145430
- FR-A1- 2 791 546
- US-A1- 2015 260 699
- US-A1- 2016 029 844

## Description

### FIELD OF THE INVENTION

This invention relates to food blenders.

### BACKGROUND OF THE INVENTION

Food blenders are widely used to make soups, drinks, sauces etc.

Customers are increasingly interested in the nutritional content of food, and the effect of different food processing operations on the nutritional value of their food.

There are known systems for analyzing the nutrient content of food items. For example, optical methods are known such as near-infrared (NIR) spectroscopy and Raman spectroscopy. These are suitable sensing approaches for use with blended food in a blender.

One feature of an optical sensing approach based on reflection is the use of a reference reflector. Thus, it is known to use a white reference for the purposes of calibration and reflection compensation for the liquid being analyzed.

Blended food is intended to be uniform and homogeneous, and is thus ideal for optical sensing. However, the blending process generates a lot of foam on the top of the blending vessel, and only the food at the bottom can reflect the true properties of the original food.

Hence, it is better to perform optical sensing at the bottom of the vessel. Due to the narrow space within the lowermost part of the vessel, it is difficult to implement a blender with integrated sensing functionality. In particular, to implement such optical sensing technology in a blender, the structure of the blender has to be redesigned to incorporate the optical sensor as well as the reference reflector.

By way of example, US 7 477 397 discloses a self-calibrating optical probe which makes use of a white reference, and is suitable for use with a blender. The probe is designed to be mounted in a hole cut in a chamber which contains the material to be analyzed. This does not allow an integrated device to be formed.

FR 2791546 A1 discloses an appliance in which bowl (1) stands on base (4) housing electric motor (6) driving rotary tool (5) mounted on shaft passing through base. Red (16) and green (17) LED light sources, inset in base top, indicate state of readiness of appliance; when bowl is on base emissions penetrate bowl's translucent material, giving diffused glow, enhanced by external ribs on bowl walls. Base top also has emitting diode (10) directing modulated beam up light guide (12) embedded in bowl's handle (3). If cover is correctly fitted, beam is redirected by reflecting underside (15) of tab (14) on cover, projecting over handle, down second guide (13) in latter, and detected by optical sensor (11) next emitter. Emitter and detector are directly connected to appliance supply lead (7). Detector controls interrupter (8) in motor supply circuit and closes it only if optical check beam is received; in that case bowl indicating glow also turns from red to green, and user can switch on motor. Opt., one or both light guides, which are e.g. 3mm dia. optic fibres, may be routed via bowl wall.

US 2015/0260699 discloses nutritional substance systems and methods which enable the tracking and communication of changes in nutritional, organoleptic, and aesthetic values of nutritional substances, and further enable the adaptive storage and adaptive conditioning of nutritional substances.

US 2016/0029844A1 discloses a system for food preparation, including a system body, a blade actuator retained by the system body; a set of blades; a blade platform rotatably mounting the set of blades, the blade platform pivotally connnected along a first edge to the system body and operable between an engaged position and a disengaged position; and a container platform pivotally connected along a first edge to the system body and operable between a loading position and a processing position.

WO 2016/072203A1 discloses an electric food processor (1) which, by operating a suction unit (45) while performing detection with a food fragment detection unit (11), prevents loss of nutritional components due to oxidation of the food and prevents the food fragments from being sucked into the suction unit (45). This electric food processor (1) is characterized by being provided with a container (2), a processing member (3) which prepares the food contained in the container (2), a main body (4) which incorporates a drvie unit (44) and the suction unit (45) and in which the container (2) is detachably mounted, a lid body (5) having a discharge nozzle unit (51a), a food fragment detection unit (11) which detects the flow of food fragments towards the discharge nozzle unit (51a), and a control unit (13) which controls operation of the drive unit (44) and the suction unit (45) depending on the detection results of the food fragment detection unit (11), wherein the control unit (13) stops operation of at least the suction unit (45) if the food fragment detection unit (11) detects a flow of food fragments towards the discharge nozzle unit (51a).

WO 2016/145430A1 discloses a blending system for displaying information. The blending system may include a display system. The display system may project or display an image on a blending container. The image may be a gradient marking or text relating to a blending process. The display system may contain a projection device capable of projecting an image, a control component operatively controlling the blender display system and at least one sensor operatively evaluating data associated with a blender device to provide feedback to the control component.

It would be desirable to enable an optical analysis system to be integrated internally within a blender without requiring a significant increase in size and still enabling reliable analysis to be carried out.

### SUMMARY OF THE INVENTION

The invention is defined by the claims.

According to examples in accordance with an aspect of the invention, there is provided a food blender, comprising:
a vessel for receiving food for blending;
a blade assembly adapted in use to be mounted at a base of the vessel, the blade assembly comprising a rotatable blade and non-rotatable blade support; and
an optical analysis system comprising:
   a sensor module having a light source and a light sensor for sensing reflected light; and
   a reference reflector,
wherein at least one of the sensor module and the reference reflector is mounted on the rotatable blade.

The optical analysis system is in this way mounted at the base of the vessel with the blades. This provides a best location for optical analysis since this area is clear of foam. There is also food in this area no matter how small the volume of food that is being blended. By using the blade as a mounting for at least part of the optical analysis system, an efficient use of space is ensured.

In a first set of examples, the sensor module and the reference reflector may both be mounted on the rotatable blade.

This means that no lateral space needs to be taken up by the optical analysis system, and it can be mounted as a single unit on the blade.

The rotatable blade for example comprises a central hub and a set of radially extending blades, wherein the sensor module and the reference reflector are mounted on an upper surface of the central hub.

This location faces into the food but does not disturb the blending function of the blades.

The sensor module and the reference reflector may be mounted aligned with the axis of rotation of the rotatable blade.

In this way, a rotationally balanced system is formed.

In a second set of examples, the sensor module is mounted on the blade support and the reference reflector is mounted on the underside of the rotatable blade or formed by the rotatable blade.

This provides the optical analysis system below the blade. This is even lower in the vessel and also provides some protection to the optical analysis system.

The blender may further comprise a controller and a motor, wherein the controller is adapted to control the motor and sensor module to align the reference reflector and the light sensor and to perform a sensing function at the end of the blending process.

This ensures reliable sensing when blending is complete, and requires only a small reflector on one or more of the blades. The blades are positioned at a suitable position for the sensing function by the controller. The reflector may even simply comprise the underside surface of a suitably designed blade.

In a third set of examples, the rotatable blade again comprises a central hub and a set of radially extending blades, and wherein the reference reflector is mounted on an underside of the central hub.

In all examples, the blender may further comprise a controller, which is adapted to control the sensor module to perform a sensing function at the end of the blending process. The reference reflector preferably comprises a white reflector. This is used for calibration of the optical analysis system, which is based on analysis of optical reflections.

A white reflector is used because it has high reflectance (close to 1) for all optical frequencies. Polytetrafluoroethylene (PTFE) and barium sulfate are ideal reflector materials, and they can reflect more than 95% incident light for both visible and near infrared light.

A spacing between the sensor module and the reference reflector is for example in the range 0.1 cm to 2 cm, for example 0.5 cm to 1 cm. This provides a compact optical arrangement.

The optical analysis system is for example adapted to obtain nutritional information relating to the food received in the vessel. In this way, the blender enables nutrient content analysis, to assist users in controlling their food intake for example for health control or dieting.

The optical analysis system may comprise a near infrared sensing system. Compared with medium infrared sensing system or other infrared sensing systems, the near infrared sensing system is cheaper and easier to implement.

Alternatively, the optical analysis system may comprise a Raman spectroscopy system.

The invention also provides a food blending method, comprising:
blending food in a vessel using a blade assembly mounted at a base of the vessel, the blade assembly comprising a rotatable blade and non-rotatable blade support; and
analyzing the blended food using an optical analysis system which comprises a sensor module having a light source and a light sensor for sensing reflected light and a reference reflector,
wherein at least one of the sensor module and the reference reflector is mounted on the rotatable blade.

The analyzing for example takes place when the blending is complete so that the blended food settles to the bottom of the vessel, where the optical analysis system is located.

### BRIEF DESCRIPTION OF THE DRAWINGS

Examples of the invention will now be described in detail with reference to the accompanying drawings, in which:
- Fig. 1: shows a blender;
- Fig. 2: shows the blender blade in more detail;
- Fig. 3: shows a first example of optical sensor configuration;
- Fig. 4: shows a second example of optical sensor configuration; and
- Fig. 5: shows a blending method.

### DETAILED DESCRIPTION OF THE EMBODIMENTS

The invention provides a food blender having an optical analysis system comprising a sensor module having a light source and a light sensor for sensing reflected light, and a reference reflector. At least one of the sensor module and the reference reflector is mounted on a rotatable blade of the food blender at the base of the vessel of the blender. This provides a best location for optical analysis since this area is clear of foam. By using the blade as a mounting for at least part of the optical analysis system, an efficient use of space is ensured.

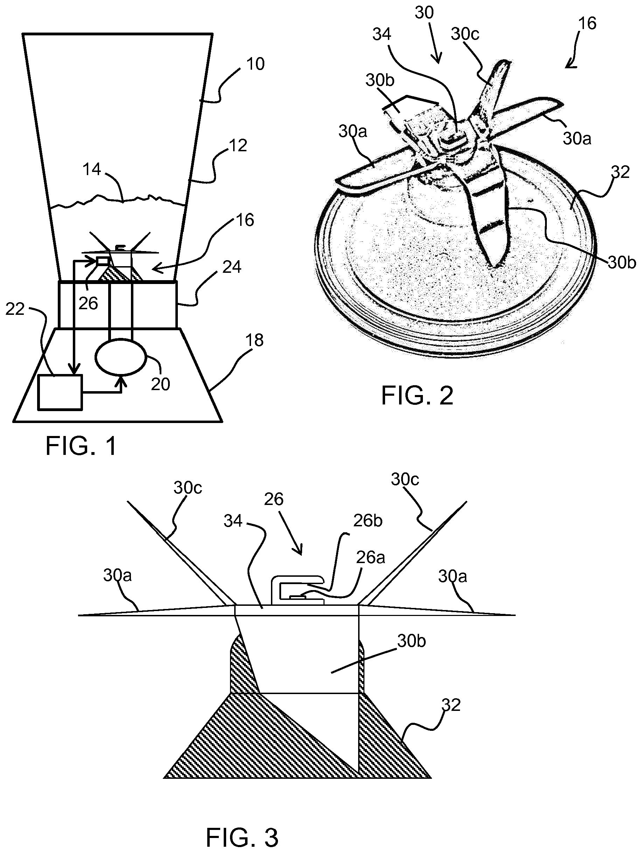

Fig. 1 shows a food blender 10 comprising a vessel 12 for receiving food 14 for blending. A blade assembly 16 is mounted at a base of the vessel (in the orientation in which the blending is to take place) and includes a rotatable blade.

The vessel is mounted on a base 18 which houses a motor 20 and a controller 22. In one example the vessel is detachable from the base, and is then turned over so that the base of the vessel 12 becomes the top of the vessel when it is to be used as a container.

The blade assembly is for example part of a lid 24 which fits to the vessel and provides an interface to the motor 20.

The blender further comprises an optical analysis system 26 comprising a sensor module having a light source and a light sensor for sensing reflected light. The system 26 also has a reference reflector for calibration purposes.

As shown in simplified form in Fig. 1, the optical analysis system is at least partly mounted to the rotatable blade.

Fig. 2 shows an example of the blade assembly 16, comprising a rotatable blade 30 and a non-rotatable blade support 32. In this example, the blade 30 has horizontal blade portions 30a, downwardly directed blade portions 30b and upwardly directed blade portions 30c. However different blade designs are possible. The blade assembly has a central hub 34 from which the blade portions project radially outwardly.

The optical analysis system 26 is thus mounted at the base of the vessel with the blades. This provides a best location for optical analysis since this area is clear of foam. A small volume of food can also be analyzed.

Fig. 3 shows a first example of the optical analysis system in more detail.

The optical analysis system comprises a sensor module 26a and a reference reflector 26b. They are both mounted on the rotatable blade. In this example, they are mounted on the top surface, i.e. facing into the main vessel volume, of the central hub 34. The sensor module faces the reflector, with a fixed spacing, for example of 0.1 cm to 2 cm, for example 0.5 cm to 1 cm. This provides a compact optical arrangement.

The optical analysis system is mounted over the rotation axle, so that the system rotates with the blades. The system does not interfere with the blending function of the blades.

The electrical connection to the sensor module passes along the rotation axis, with an electrical connection which allows relative rotation to the sensor module.

Light is emitted from a light source of the sensor module to the blended food specimen that resides in the fixed spacing. The light sensor of the sensor module then collects the reflected beam to calculate the absorbance which is related to the food nutrient content.

A small fixed gap not only enables miniaturization but also helps to prevent light leakage in a dilute specimen. If the gap size is too large, the light may scatter out from the side of the gap. If the gap size is too small, it may become too small for a diffusive reflection regime.

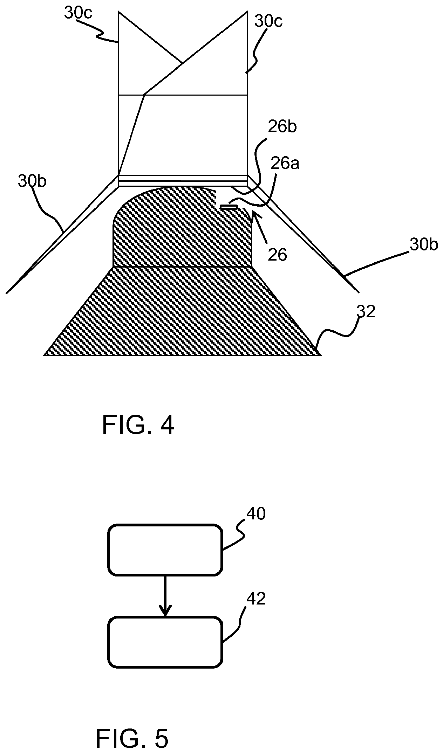

Fig. 4 shows a second example in which the sensor module 26a is mounted on the blade support 32 and the reference reflector 26b is mounted on the underside of the rotatable blade or formed by the rotatable blade.

This provides the optical analysis system below the blade. This is even lower in the vessel and also provides some protection to the optical analysis system. Furthermore, this arrangement does not require any rotational electrical connections.

In the example shown, the reference reflector is formed by the underside of the central hub 34. It may be a component attached to the blade assembly or it may be formed by the blade assembly itself.

In this case, the reference reflector 26b may rotate very fast (for example 18,000 rpm to 24,000 rpm) relative to the sensor module 26a which is kept still at the base. An optical scan for example takes 0.1 to 1 second, and hence there may be synchronization issues. To avoid any such issues, the sample can be analyzed when the blending process ends.

For this purpose, the controller 22 (which controls the motor 20) may control the sensor module 26 to perform a sensing function at the end of the blending process.

In another example, the reference reflector 26b may be mounted on the underside of one of the rotating blades, or it may be defined by the under surface of one or all of the rotating blades. The lowest blades 30b may be used for this purpose. If there are no downwardly pointing blades 30b, the horizontal blades may then be used.

In this case, the controller 22 may control the motor to align the reference reflector and the light sensor at the end of the blending process, and also to perform the sensing function at the end of the blending process in the correctly aligned position.

This ensures reliable sensing when blending is complete, and requires only a small reflector on one or more of the blades.

The optical analysis system is used to obtain nutrient information relating to the food received in the vessel. In this way, the blender enables nutrient content analysis, to assist users in controlling their food intake for example for health control or dieting.

The optical analysis system may comprise a near infrared sensing system or a Raman spectroscopy system, or any other optical system based on the analysis of a reflected optical system.

The white reflector is for example formed from Polytetrafluoroethylene (PTFE) or barium sulfate. To meet the food grade standards, a silica glass cover may be used to prevent direct contact between the reference reflector and the blended food.

Fig. 5 shows a food blending method, comprising:
in step 40, blending food in a vessel using the blade assembly mounted at the base (when in the blending position) of the vessel; and
in step 42, analyzing the blended food using the optical analysis system.

The analyzing for example takes place when the blending is complete so that the blended food settles to the bottom of the vessel, where the optical analysis system is located.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

Any reference signs in the claims should not be construed as limiting the scope.

## Claims

1. A food blender (10), comprising:
a vessel (12) for receiving food for blending;
a blade assembly (16) adapted in use to be mounted at a base of the vessel, the blade assembly comprising a rotatable blade (30) and non-rotatable blade support (32); and
an optical analysis system (26) comprising:
a sensor module (26a) having a light source and a light sensor for sensing reflected light; and **characterised by**
a reference reflector (26b),
wherein at least one of the sensor module (26a) and the reference reflector (26b) is mounted on the rotatable blade (30).

2. A food blender as claimed in claim 1, wherein the sensor module (26a) and the reference reflector (26b) are both mounted on the rotatable blade.

3. A food blender as claimed in claim 2, wherein the rotatable blade (30) comprises a central hub (34) and a set of radially extending blades (30a, 30b, 30c), wherein the sensor module and the reference reflector are mounted on an upper surface of the central hub (34).

4. A food blender as claimed in claim 3, wherein the sensor module and the reference reflector are mounted aligned with the axis of rotation of the rotatable blade (30).

5. A food blender as claimed in claim 1, wherein the sensor module (26a) is mounted on the blade support (32) and the reference reflector (26b) is mounted on the underside of the rotatable blade or formed by the rotatable blade (30).

6. A food blender as claimed in claim 5, further comprising a controller (22) and a motor (20), wherein the controller is adapted to control the motor and sensor module to align the reference reflector and the light sensor and to perform a sensing function at the end of the blending process.

7. A food blender as claimed in claim 5, wherein the rotatable blade comprises a central hub (34) and a set of radially extending blades (30a, 30b, 30c), wherein the reference reflector (26b) is mounted on an underside of the central hub (34).

8. A food blender as claimed in any preceding claim, further comprising a controller (22), which is adapted to control the sensor module to perform a sensing function at the end of the blending process.

9. A food blender as claimed in any one of claims 1 to 7, wherein the reference reflector (26b) comprises a white reflector.

10. A food blender as claimed in any one of claims 1 to 7, wherein a spacing between the sensor module (26a) and the reference reflector (26b) is in the range 0.1 cm to 2 cm.

11. A food blender as claimed in any one of claims 1 to 7, wherein the optical analysis system is adapted to obtain nutrient information relating to the food (14) received in the vessel (12).

12. A food blender as claimed in any one of claims 1 to 7, wherein the optical analysis system comprises a near infrared sensing system.

13. A food blender as claimed in any one of claims 1 to 7, wherein the optical analysis system comprises a Raman spectroscopy system.

14. A food blending method, comprising:
(40) blending food in a vessel using a blade assembly mounted at a base of the vessel, the blade assembly comprising a rotatable blade and non-rotatable blade support; and **characterised by**
(42) analyzing the blended food using an optical analysis system which comprises a sensor module having a light source and a light sensor for sensing reflected light and a reference reflector,
wherein at least one of the sensor module and the reference reflector is mounted on the rotatable blade.

15. A method as claimed in claim 14, wherein the analyzing takes place when the blending is complete.

## Patentansprüche

1. Lebensmittelmixer (10), umfassend:
ein Gefäß (12) zum Aufnehmen von Lebensmittel für Mischung;
eine Klingenbaugruppe (16), die bei Anwendung zur Montage an einer Basis des Gefäßes geeignet ist, wobei die Klingenbaugruppe eine drehbare Klinge (30) und einen nicht drehbaren Klingenträger (32) aufweist; und
ein optisches Analysesystem (26), umfassend:
ein Sensormodul (26a) mit einer Lichtquelle und einem Lichtsensor zum Erfassen von reflektiertem Licht; und **gekennzeichnet durch**
einen Referenzreflektor (26b),
wobei mindestens eines von dem Sensormodul (26a) und dem Referenzreflektor (26b) an der drehbaren Klinge (30) angebracht ist.

2. Lebensmittelmixer nach Anspruch 1, wobei das Sensormodul (26a) und der Referenzreflektor (26b) beide an der drehbaren Klinge angebracht sind.

3. Lebensmittelmixer nach Anspruch 2, wobei die drehbare Klinge (30) eine zentrale Nabe (34) und einen Satz radial erstreckender Klingen (30a, 30b, 30c) aufweist, wobei das Sensormodul und der Referenzreflektor auf einer oberen Fläche der zentralen Nabe (34) angebracht sind.

4. Lebensmittelmixer nach Anspruch 3, wobei das Sensormodul und der Referenzreflektor zum Ausrichten zu einer Drehachse der drehbaren Klinge (30) angebracht sind.

5. Lebensmittelmixer nach Anspruch 1, wobei das Sensormodul (26a) am Klingenträger (32) angebracht ist, und der Referenzreflektor (26b) an der Unterseite der drehbaren Klinge angebracht oder von der drehbaren Klinge(30) gebildet ist.

6. Lebensmittelmixer nach Anspruch 5, ferner umfassend eine Steuerung (22) und einen Motor (20), wobei die Steuerung dazu geeignet ist, der Motor und das Sensormodul derart zu steuern, dass der Referenzreflektor und der Lichtsensor ausgerichtet sind und eine Erfassungsfunktion am Ende des Mischvorgangs durchgeführt wird.

7. Lebensmittelmixer nach Anspruch 5, wobei die drehbare Klinge eine zentrale Nabe (34) und einen Satz radial erstreckender Klingen (30a, 30b, 30c) aufweist, wobei der Referenzreflektor (26b) an einer Unterseite der zentrale Nabe (34) angebracht ist.

8. Lebensmittelmixer nach einem der vorhergehenden Ansprüche, ferner umfassend eine Steuerung (22), die dazu geeignet ist, das Sensormodul derart zu steuern, dass eine Erfassungsfunktion am Ende des Mischvorgangs durchgeführt wird.

9. Lebensmittelmixer nach einem der Ansprüche 1 bis 7, wobei der Referenzreflektor (26b) einen weißen Reflektor umfasst.

10. Lebensmittelmixer nach einem der Ansprüche 1 bis 7, wobei ein Abstand zwischen dem Sensormodul (26a) und dem Referenzreflektor (26b) im Bereich von 0,1 cm bis 2 cm liegt.

11. Lebensmittelmixer nach einem der Ansprüche 1 bis 7, wobei das optische Analysesystem dazu geeignet ist, Nährstoffinformationen über das in dem Gefäß (12) aufgenommene Lebensmittel (14) zu erhalten.

12. Lebensmittelmixer nach einem der Ansprüche 1 bis 7, wobei das optische Analysesystem ein Nah-Infrarot-Erfassungssystem umfasst.

13. Lebensmittelmixer nach einem der Ansprüche 1 bis 7, wobei das optische Analysesystem ein Raman-Spektroskopiesystem umfasst.

14. Verfahren zum Mischen von Lebensmittel, umfassend:
Mischen (40) von Lebensmittel in einem Gefäß unter Verwendung einer Klingenbaugruppe, die an einer Basis des Gefäßes angebracht ist, wobei die Klingenbaugruppe eine drehbare Klinge und einen nicht drehbaren Klingenträger aufweist; und **gekennzeichnet durch**
Analysieren (42) des gemischten Lebensmittels unter Verwendung eines optischen Analysesystems, das ein Sensormodul mit einer Lichtquelle und einem Lichtsensor zum Erfassen von reflektiertem Licht und einen Referenzreflektor umfasst,
wobei mindestens eines von dem Sensormodul und dem Referenzreflektor an der drehbaren Klinge angebracht ist.

15. Verfahren nach Anspruch 14, wobei das Analysieren statt findet, wenn das Mischen abgeschlossen ist.

## Revendications

1. Mélangeur d'aliments (10), comprenant:
un récipient (12) pour recevoir des aliments à mélanger;
un ensemble lame (16) adapté en utilisation pour être monté à une base du récipient, l' ensemble lame comprenant une lame rotative (30) et un support de lame non rotatif (32); et
un système d'analyse optique (26) comprenant:
un module de capteur (26a) ayant une source de lumière et un capteur de lumière pour détecter la lumière réfléchie; et **caractérisé par**
un réflecteur de référence (26b),
dans lequel au moins l'un du module capteur (26a) et le réflecteur de référence (26b) est monté sur la lame rotative (30).

2. Mélangeur d'aliments selon la revendication 1, dans lequel le module de capteur (26a) et le réflecteur de référence (26b) sont tous montés sur la lame rotative.

3. Mélangeur d'aliments selon la revendication 2, dans lequel la lame rotative (30) comprend un moyeu central (34) et un ensemble de lames s'étendant radialement (30a, 30b, 30c), dans lequel le module de capteur et le réflecteur de référence sont montés sur une surface supérieure du moyeu central (34).

4. Mélangeur d'aliments selon la revendication 3, dans lequel le module de capteur et le réflecteur de référence sont montés alignés avec l'axe de rotation de la lame rotative (30).

5. Mélangeur d'aliments selon la revendication 1, dans lequel le module de capteur (26a) est monté sur le support de lame (32) et le réflecteur de référence (26b) est monté sur le dessous de la lame rotative ou formé par la lame rotative (30).

6. Mélangeur d'aliments selon la revendication 5, comprenant en outre un contrôleur (22) et un moteur (20), dans lequel le contrôleur est adapté pour contrôler le moteur et le module de capteur pour aligner le réflecteur de référence et le capteur de lumière et pour effectuer une fonction de détection à la fin du processus de mélange.

7. Mélangeur d'aliments selon la revendication 5, dans lequel la lame rotative comprend un moyeu central (34) et un ensemble de lames s'étendant radialement (30a, 30b, 30c), dans lequel le réflecteur de référence (26b) est monté sur le dessous de le moyeu central (34).

8. Mélangeur d'aliments selon l'une quelconque des revendications précédentes, comprenant en outre un contrôleur (22), qui est adapté pour contrôler le module de capteur pour effectuer une fonction de détection à la fin du processus de mélange.

9. Mélangeur d'aliments selon l'une quelconque des revendications 1 à 7, dans lequel le réflecteur de référence (26b) comprend un réflecteur blanc.

10. Mélangeur d'aliments selon l'une quelconque des revendications 1 à 7, dans lequel un espacement entre le module de capteur (26a) et le réflecteur de référence (26b) est dans la plage de 0.1 cm à 2 cm.

11. Mélangeur d'aliments selon l'une quelconque des revendications 1 à 7, dans lequel le système d'analyse optique est adapté pour obtenir des informations nutritionnelles concernant l'aliment (14) reçu dans le récipient (12).

12. Mélangeur d'aliments selon l'une quelconque des revendications 1 à 7, dans lequel le système d'analyse optique comprend un système de détection proche infrarouge.

13. Mélangeur d'aliments selon l'une quelconque des revendications 1 à 7, dans lequel le système d'analyse optique comprend un système de spectroscopie Raman.

14. Procédé de mélanger d'aliments, comprenant:
(40) mélanger d'aliments dans un récipient en utilisant un ensemble lame monté à une base du récipient, l'ensemble lame comprenant une lame rotative et un support de lame non rotatif; et **caractérisé par**
(42) analyser l'aliment mélangé en utilisant un système d'analyse optique qui comprend un module de capteur ayant une source de lumière et un capteur de lumière pour détecter la lumière réfléchie et un réflecteur de référence,
dans lequel au moins l'un du module de capteur et le réflecteur de référence est monté sur la lame rotative.

15. Procédé selon la revendication 14, dans lequel l'analyse a lieu lorsque le mélange est complété.
